(19) Europäisches Patentamt
European Patent Office
Office européen des brevets

(11) Numéro de publication: **0 399 901 A1**

(12) **DEMANDE DE BREVET EUROPEEN**

(21) Numéro de dépôt: **90401368.7**

(51) Int. Cl.[5]: **C07D 233/94**

(22) Date de dépôt: **22.05.90**

(30) Priorité: **23.05.89 FR 8906703**

(43) Date de publication de la demande: **28.11.90 Bulletin 90/48**

(84) Etats contractants désignés: **AT BE CH DE DK ES FR GB GR IT LI LU NL SE**

(71) Demandeur: **RHONE-POULENC SANTE**
**20, avenue Raymond Aron**
**F-92160 Antony(FR)**

(72) Inventeur: **Massonneau, Viviane**
**Charrière Blanche Le Tilleul**
**F-69130 Ecully(FR)**
Inventeur: **Mulhauser, Michel**
**Immeuble "Frênes 4" Résidence Charrière Blanche**
**F-69130 Ecully(FR)**

(74) Mandataire: **Pilard, Jacques et al**
**RHONE-POULENC SANTE, Service Brevets,**
**20 Avenue Raymond Aron**
**F-92165 Antony Cédex(FR)**

(54) **Procédé de préparation du (chloro-3 hydroxy-2-propyl)-1 méthyl-2 nitro-5 imidazole.**

(57) Procédé de préparation du (chloro-3 hydroxy-2 propyl)-1 méthyl-2 nitro-5 imidazole par action du sulfate de chloro-3 propanediol-1,2 sur un dérivé de l'imidazole de formule générale :

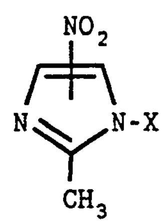

(X représente un atome d'hydrogène ou un radical éliminable par hydrolyse acide ou par alcoolyse), suivie de l'hydrolyse ou de l'alcoolyse du produit obtenu.

EP 0 399 901 A1

# PROCEDE DE PREPARATION DU (CHLORO-3 HYDROXY-2 PROPYL)-1 METHYL-2 NITRO-5 IMIDAZOLE

La présente invention concerne un nouveau procédé de préparation du (chloro-3 hydroxy-2 propyl)-1 méthyl-2 nitro-5 imidazole.

Le (chloro-3 hydroxy-2 propyl)-1 méthyl-2 nitro-5 imidazole (ou ornidazole) présente des propriétés anti-protozoaires et anti-bactériennes particulièrement intéressantes.

Il est connu de préparer le (chloro-3 hydroxy-2 propyl)-1 méthyl-2 nitro-5 imidazole à partir du méthyl-2 nitro-4 (ou -5) imidazole soit par action du sulfate de bis-(chloro-3 hydroxy-2 propyl) en passant intermédiairement par l'(époxy-2,3 propyl)-1 méthyl-2 nitro-5 imidazole (brevets américains US 3 493 582 et US 3 519 637), soit par action de l'épichlorhydrine en présence d'acide de Lewis [E. Alcade et coll., J. Het. Chem., 21, 1647 (1984)]. Cependant la mise en oeuvre de ces procédés ne permet pas toujours d'obtenir des rendements satisfaisants.

Il a maintenant été trouvé, et c'est ce qui fait l'objet de la présente invention, que le (chloro-3 hydroxy-2 propyl)-1 méthyl-2 nitro-5 imidazole peut être obtenu avec de bons rendements par action du sulfate de chloro-3 propanediol-1,2 de formule :

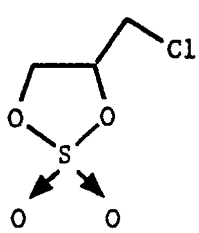

(I)

sur un dérivé de l'imidazole de formule générale :

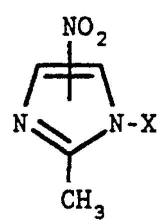

(II)

dans laquelle X représente un atome d'hydrogène ou un radical éliminable par hydrolyse ou alcoolyse, tel qu'un radical hydroxyméthyle, alcoxyméthyle dont la partie alcoyle contient 1 à 4 atomes de carbone, acyloxyméthyle dont la partie acyle contient 1 à 4 atomes de carbone ou un radical éthylénique allylique, tel que le radical allyle, ou un radical arylméthyle, tel que le radical benzyle suivie de l'hydrolyse acide ou de l'alcoolyse du produit de condensation ainsi obtenu.

La condensation du sulfate cyclique de formule (I) sur le dérivé de l'imidazole de formule générale (II) est effectuée à une température comprise entre 60 et 120°C en présence éventuellement d'un solvant organique choisi parmi les esters tel que l'acétate de méthyle, l'acétate d'éthyle ou le diacétate de glycol, les cétones telles que la méthylisobutylcétone, les éthers tels que le méthyltertiobutyléther, les hydrocarbures aliphatiques ou aromatiques éventuellement halogénés tels que le benzène, le toluène, le xylène, le chloroforme, le dichlorométhane ou le chlorobenzène ou les nitriles tels que l'acétonitrile.

Le produit de condensation qui précipite peut être solubilisé :
- soit dans une solution aqueuse d'un acide minéral fort tel que, par exemple, l'acide sulfurique ou l'acide chlorhydrique
- soit dans un alcool, tel que, par exemple, le méthanol ou l'éthanol.

Lorsque le produit de condensation est solubilisé dans l'eau acidifiée, le (chloro-3 hydroxy-2 propyl)-1 méthyl-2 nitro-5 imidazole est extrait selon les techniques habituelles après alcalinisation du mélange réactionnel à un pH voisin de 10.

Lorsque le produit de condensation est solubilisé dans un alcool, le (chloro-3 hydroxy-2 propyl)-1 méthyl-2 nitro-5 imidazole est isolé selon les techniques habituelles sans traitement préalable du mélange réactionnel.

Pour la mise en oeuvre du procédé, il n'est pas nécessaire d'isoler le produit de condensation intermédiaire, l'hydrolyse ou l'alcoolyse pouvant être enchaînées dans le même appareil.

Le sulfate de chloro-3 propanediol-1,2 peut être obtenu par oxydation du sulfite correspondant.

Généralement l'oxydation est réalisée au moyen d'un hypohalogénite (hypochlorite ou hypobromite alcalin ou alcalino terreux) de préférence l'hypochlorite de sodium, de potassium ou de calcium, en présence d'une quantité catalytique d'un dérivé du ruthénium choisi de préférence parmi l'oxyde de ruthénium (IV) ($RuO_2$) et le chlorure de ruthénium ($RuCl_3$).

Le procédé peut être mis en oeuvre en milieu aqueux ou hydroorganique biphasique.

Lorsque le procédé est mis en oeuvre en milieu hydroorganique biphasique, le solvant est généralement choisi parmi les hydrocarbures aliphatiques ou cycloaliphatiques éventuellement halogénés, tels que l'hexane, le cyclohexane, le chlorure de méthylène, le chloroforme, le tétrachlorure de carbone ou le dichloroéthane, ou les esters tels que l'acétate de méthyle ou l'acétate d'éthyle.

Généralement, on utilise un excès d'hypohalogénite par rapport au sulfite de chloro-3

propanediol-1,2 mis en oeuvre. De préférence, on utilise 1 à 2 moles d'hypochlorite par mole de sulfite cyclique.

Généralement, on utilise une quantité catalytique de dérivé du ruthénium comprise entre $10^{-6}$ et $10^{-1}$ mole par mole de sulfite cyclique.

Le sulfate de chloro-3 propanediol-1,2 est séparé du mélange réactionnel selon les techniques habituelles.

Le sulfite de chloro-3 propanediol-1,2 peut être préparé selon les méthodes connues et en particulier selon le procédé décrit par D.S. Breslow et H. Skolnik, "The Chemistry of Heterocyclic Compounds - Multi-Sulphur and Sulphur and Oxygen 5- and 6- Membered Heterocycles, 1966, Part I, p. 1 et Part II, p. 663 ou par H.F. Van woerden, Chem. Rev., 63, 557 (1963).

Le dérivé de l'imidazole de formule générale (II) peut être préparé dans les conditions décrites dans le brevet anglais GB 1 026 631.

L'exemple suivant, donné à titre non limitatif, illustre le procédé selon l'invention.

## EXEMPLE

Dans un ballon muni d'un agitateur, on introduit 6,60 g d'acétoxyméthyl-1 méthyl-2 nitro-4 imidazole (0,033 mole) et 8,70 g de sulfate de chloro-3 propanediol-1,2 (0,050 mole) puis on chauffe à 110° C pendant 30 minutes. Le mélange réactionnel est solubilisé par addition de 20 cm3 de toluène. On maintient à 110° C pendant 2 heures. On ajoute alors 0,1 g d'acide sulfurique concentré (0,001 mole) et 10 cm3 de méthanol puis chauffe au reflux pendant 2 heures. On ajoute alors 10 cm3 d'eau puis poursuit encore le chauffage au reflux pendant 1 heure 30 minutes.

Après refroidissement, la phase aqueuse est séparée par décantation. L'analyse par chromatographie liquide à haute performance (CLHP) montre qu'elle contient :

- 0,415 g de méthyl-2 nitro-4 (ou -5) imidazole
- 6,06 g de (chloro-3 hydroxy-2 propyl)-1 méthyl-2 nitro-5 imidazole.

Le taux de transformation de l'acétoxyméthyl-1 méthyl-2 nitro-4 imidazole est de 90 %.

Le rendement en (chloro-3 hydroxy-2 propyl)-1 méthyl-2 nitro-5 imidazole est de 83,7 % par rapport à l'acétoxyméthyl-1 méthyl-2 nitro-4 imidazole mis en oeuvre et de 93 % par rapport à l'acétoxyméthyl-1 méthyl-2 nitro-4 imidazole transforme.

A la phase aqueuse, on ajoute une solution de soude concentrée jusqu'à ce que le pH soit égal à 8. On extrait alors par 2 fois 50 cm3 de dichlorométhane. La phase organique obtenue est lavée par 2 fois 20 cm3 d'eau puis concentrée à sec.

On obtient ainsi 6,67 g d'un produit contenant 84 % de (chloro-3 hydroxy-2 propyl)-1 méthyl-2 nitro-5 imidazole (dosage par CLHP avec étalonnage externe).

Le rendement en (chloro-3 hydroxy-2 propyl)-1 méthyl-2 nitro-5 imidazole est de 77 % par rapport à l'acétoxyméthyl-1 méthyl-2 nitro-4 imidazole mis en oeuvre.

Le sulfate de chloro-3 propanediol-1,2 peut être préparé de la manière suivante :

Dans un ballon de 250 cm3, on introduit 4,96 g de sulfite de chloro-3 propanediol-1,2 (0,03 mole) et 25 cm3 d'eau. Après refroidissement à 0° C, on ajoute, sous agitation, 22,5 cm3 d'une solution d'hypochlorite de sodium à 2 moles par litre (soit 0,045 mole) contenant 4,5 mg d'oxyde de ruthénium (IV) dihydrate (0,027 x $10^{-3}$ mole).

On poursuit l'agitation pendant 5 minutes à 2° C. On extrait le mélange réactionnel par 2 fois 20 cm3 de dichlorométhane. La phase organique est traitée par 0,5 cm3 d'isopropanol puis est lavée par 15 cm3 d'eau. Après séchage de la phase organique et concentration sous pression réduite (2 mm de mercure ; 0,47 kPa), on obtient 3,95 g de sulfate de chloro-3 propanediol-1,2 sous forme d'un liquide incolore.

Le rendement est de 76,4 %.

## Revendications

1 - Procédé de préparation du (chloro-3 hydroxy-2 propyl)-1 méthyl-2 nitro-5 imidazole caractérisé en ce que l'on fait réagir le sulfate de chloro-3 propanediol-1,2 de formule :

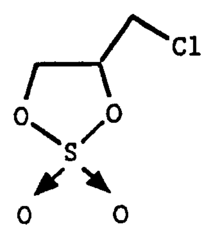

sur un dérivé de l'imidazole de formule générale :

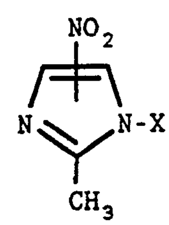

dans laquelle X représente un atome d'hydrogène ou un radical éliminable par hydrolyse acide ou alcoolyse, puis hydrolyse ou alcoolyse le produit

obtenu et isole le (chloro-3 hydroxy-2 propyl)-1 méthyl-2 nitro-5 imidazole.

2 - Procédé selon la revendication 1 caractérisé en ce que le radical éliminable par hydrolyse acide ou alcoolyse est choisi parmi les radicaux hydroxyméthyle, alcoxyméthyle dont la partie alcoyle contient 1 à 4 atomes de carbone, acyloxyméthyle dont la partie acyle contient 1 à 4 atomes de carbone ou un radical éthylénique allylique ou un radical aryméthyle.

3 - Procédé selon l'une des revendications 1 ou 2 caractérisé en ce que l'on opère dans un solvant organique choisi parmi les esters, les éthers, les cétones, les hydrocarbures aliphatiques ou aromatiques éventuellement halogénés et les nitriles.

4 - Procédé selon la revendication 3 caractérisé en ce que le solvant est choisi parmi l'acétate de méthyle, l'acétate d'éthyle, le diacétate de glycol, le méthyltertiobutyléther, la méthylisobutylcétone, le chloroforme, le dichlorométhane, le benzène, le toluène, le xylène, le chlorobenzène et l'acétonitrile.

5 - Procédé selon la revendication 1 caractérisé en ce que l'hydrolyse du produit de condensation est effectuée en présence d'un acide fort choisi parmi l'acide sulfurique et l'acide chlorhydrique.

6 - Procédé selon la revendication 1 caractérisé en ce que l'alcoolyse est effectuée par chauffage en présence d'un alcool choisi parmi le méthanol et l'éthanol.

Office européen des brevets

# RAPPORT DE RECHERCHE EUROPEENNE

Numero de la demande

EP 90 40 1368

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | US-A-3 280 139 (JOSEF KLOSA (DELMAR CHEMICALS LTD)) --- | | C 07 D 233/94 |
| D,A | US-A-3 519 637 (MAX HOFFER (HOFFMANN-LA ROCHE))) --- | | |
| D,A | US-A-3 493 582 (MAX HOFFER (HOFFMANN-LA ROCHE))) ----- | | |

| DOMAINES TECHNIQUES RECHERCHES (Int. Cl.5) |
|---|
| C 07 D 233/00 |

Le présent rapport a été établi pour toutes les revendications

| Lieu de la recherche | Date d'achèvement de la recherche | Examinateur |
|---|---|---|
| LA HAYE | 27-07-1990 | DE BUYSER I.A.F. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant